Europäisches Patentamt

European Patent Office

Office européen des brevets

(19)

(11) **EP 1 541 160 A1**

(12) **EUROPEAN PATENT APPLICATION**
published in accordance with Art. 158(3) EPC

(43) Date of publication:
    **15.06.2005 Bulletin 2005/24**

(51) Int Cl.⁷: **A61K 35/78**, A61K 38/02,
     A61P 3/04, A61P 3/06,
     A23L 1/20

(21) Application number: **03725666.6**

(22) Date of filing: **24.04.2003**

(86) International application number:
    **PCT/JP2003/005305**

(87) International publication number:
    **WO 2004/009107 (29.01.2004 Gazette 2004/05)**

(84) Designated Contracting States:
    **AT BE BG CH CY CZ DE DK EE ES FI FR GB GR
    HU IE IT LI LU MC NL PT RO SE SI SK TR**
    Designated Extension States:
    **AL LT LV MK**

(30) Priority: **19.07.2002 JP 2002211844**

(71) Applicant: **Fuji Oil Company, Ltd.
    Osaka-shi, Osaka 542-0086 (JP)**

(72) Inventors:
    • **Baba, Toshimitsu, c/o Fuji Oil Company, Ltd
      Izumisano-shi, Osaka 598-8540 (JP)**
    • **Hirotsuka, Motohiko, c/o Fuji Oil Company, Ltd.
      Izumisano-shi, Osaka 598-8540 (JP)**

    • **Miyazaki, Chiaki, c/o Fuji Oil Company, Ltd
      Izumisano-shi, Osaka 598-8540 (JP)**
    • **Okajima, Tetsuhiko, c/o Fuji Oil Company, Ltd.
      Izumisano-shi, Osaka 598-8540 (JP)**
    • **KAMBARA, Hirofumi
      Shizuoka-shi, Shizuoka 420-0844 (JP)**
    • **KITO, Makoto
      Kyoto-shi, Kyoto 606-0803 (JP)**

(74) Representative: **Hayes, Adrian Chetwynd et al
    Boult Wade Tennant,
    Verulam Gardens
    70 Gray's Inn Road
    London WC1X 8BT (GB)**

(54) **BODY FAT PERCENTAGE-LOWERING AGENT OR BODY FAT PERCENTAGE INCREASE INHIBITOR**

(57)    A body fat percentage-lowering agent or an inhibitor for an increase in body fat percentage containing soybean 7S protein as the active ingredient and foods containing the same. As the soybean protein 7S protein, use is made of a protein the purity of which is elevated by sufficiently separating from 11S globulin, preferably a protein having reduced contents of polar lipids and phytic acid.

EP 1 541 160 A1

# EP 1 541 160 A1

## Description

Technical Field

The present invention relates to an agent lowering body fat percentage or an inhibitor of body fat percentage increase containing soybean 7S protein as an active ingredient, and to foodstuffs using the same.

Background Art

While soybean protein is excellent as nutrients among plant proteins, it is considered to be food materials noticed as a physiologically functioning material in recent years since various physiological effects have been found in the soybean protein. For example, Iritani et al. showed that the soybean protein can lower neutral fat by suppressing the activity of fatty acid synthesis enzymes in the liver (J. Nutr., 126, 380, 1996). The effect of each of the total soybean globulin, 7S globulin and 11S globulin on the lipids in the blood and liver has been investigated, and the proteins have been shown to be totally excellent in the ability of lowering cholesterol and neutral fat concentration in the blood as compared with casein as an animal protein (Okita et al., J. Nutr., 27, 379, 1981).

On the other hand, the some proteins derived from soybean have affinity with polar lipids which constitute membranes such as a plasma membrane, a membrane of protein body and oil body. The protein is named as "oil body-associating protein" by Samoto et al. The "oil body-associating protein" is a collective name of a group of proteins comprising membrane proteins as a main component. Especially, the protein is a fraction containing proteins with estimated molecular weights of 34 kDa, 24 kDa and 18 kDa as measured by SDS-polyacrylamide gel electrophoresis and containing about 10% by weight of polar lipids extracted with polar solvents that is a mixture of chloroform and ethanol in a ratio of 2:1. Samoto et al reported that such proteins account for about 35% of soybean protein isolate that is industrially produced (Biosci. Biotechnol. Biochem., 62(5), 935-940 1998)). The oil body-associating protein has poor flavor and high allergenicity.

However, the content of the oil body-associating protein has been estimated to be much lower than the real content or has not been taken into consideration when assayed in conventional SDS-polyacrylamide gel electrophoresis that is commonly used in the assay of the protein composition of the soybean protein, because the oil body-associating protein can be hardly stained.

As described above, while effects of soybean 7S globulin on lowering the cholesterol and neutral fat level in the blood have been evaluated, there are no reports of body fat percentage-lowering effects and body fat increase inhibiting effects by using soybean 7S globulin.

The inventors of the present invention found that the percentage of the body fat can be lowered or its increase can be inhibited by intake of soybean 7S protein containing large amounts of soybean 7S globulin after fractionating 7S protein from soybean protein, and have completed the present invention.

Therefore, the problem of the present invention is to provide an agent lowering body fat percentage or an inhibitor of body fat percentage increase containing soy bean 7S protein as an active ingredient and to provide foodstuffs containing the same obtained by 7S protein containing large amounts of soybean 7S globulin from soybean protein.

Disclosure of Invention

The present invention provides an agent lowering body fat percentage or an inhibitor of body fat percentage increase containing soybean 7S protein as an active ingredient. The effect of the present invention is to inhibit the body fat increase as well as to lower the body fat actively in overweight persons with the body fat percentage of 30% or more. The present invention also provides foodstuffs containing the agent lowering body fat percentage or the inhibitor of body fat percentage increase.

The present invention provides:

(1) an agent lowering body fat percentage or an inhibitor of body fat percentage increase containing soybean 7S protein as an active ingredient;
(2) the agent lowering body fat percentage according to (1) for overweight persons with a body fat percentage of 30% or more;
(3) the agent lowering body fat percentage or the inhibitor of body fat percentage increase according to any one of (1) and (2) containing 2.5% or less of polar lipids in a solid content of soybean 7S protein;
(4) the agent lowering body fat percentage or the inhibitor of body fat percentage increase according to any one of (1) and (2) containing 1.2% or less of phytic acid in a solid content of soybean 7S protein; and
(5) foodstuffs containing the agent lowering body fat percentage or the inhibitor of body fat percentage increase according to (1).

Best Mode for Carrying Out the Invention

In the present invention, soybean 7S protein refers to a protein containing large amounts of soybean 7S globulin. Generally, soybean 7S globulin refers to a protein having a molecular weight represented by an ultracentrifuge sedimentation coefficient corresponding to 7S among the globulin as a collective name of soluble globular protein. The globulin includes 2S, 7S, 11S and 15S proteins depending on its molecular weight distribution, and 7S and 11S proteins are known to be contained in storage proteins of leguminous plant such as soybeans in large quantities. Incidentally, soybean 7S globulin substantially corresponds to β-conglycinin in an immunological nomenclature.

The following known method for removing 11S globulin may be employed to obtain soybean 7S protein in the present invention. Examples of the method available include any one of Thahn-Shibasaki method (Thahn, V. H. and Shibasaki, K., J. Agric. Food Chem., 24, 117, 1976), cold-insoluble fraction (CIF) by crio-precipitation method (Briggs, D. R and Mann, R. L., Cereal Chem., 27, 243, 1950), and a fractionation method by adding 0.1 N calcium chloride (Wolf, W. J. and Sly, D. A., Cereal Chem., 44, 653, 1967). Bred soybean deficient in 11S globulin fraction may be also used as disclosed in USP No. 6171640.

After removing 11S globulin by any one of the methods above, 7S protein can be prepared by purification by isoelectric precipitation, neutralization and sterilization thereafter. In this case, 7S protein with purity sufficient for various uses may be fractionated without using any reducing agents, and the protein containing no reducing agent may be expected to be widely applicable as the agent lowering body fat percentage or the inhibitor of body fat percentage increase.

The content of 7S globulin as the purity of the 7S protein (based on SPE standard to be described below) is 40% or higher, preferably 60% or higher, more preferably 80% or higher, and most preferably 90% or higher. The expected effect may be obtained by intake of smaller quantity of the protein as the purity is higher.

Increasing separation efficiency from 11S globulin may afford high purity 7S protein. An example of the method comprises heating a solution containing the soybean protein under a slightly acidic condition, and fractionating the protein into a soluble fraction abundant in 7S protein and an insoluble fraction abundant in 11s protein at pH 5.6 to 6.6. This method provides high purity 7S protein containing 1% or less of the polar lipid as an index of the oil body-associating protein in the solid content. It is also effective to remove phytic acid by decomposition with an enzyme such as phytase before separation of 11S globulin. This method affords high purity 7s protein with a low percentage of phytic acid having the phytic acid content of 1.2% or less, usually 0.2% or less in the solid content. Separation efficiency may be further improved by using the both methods together.

The body fat percentage as a measure of obesity differs depending on sex and age. A male with a body fat percentage of 30% and 35% are considered to be obesity and extreme obesity, respectively, while a female with a body fat percentage of 30%, 35% and 40% are considered to be slight obesity, obesity and extreme obesity, respectively. The agent lowering body fat percentage of the present invention is suitable for an overweight person with a body fat percentage of 30% or higher, preferably effective for lowering the percentage of the body fat in an overweight person with a body fat percentage of 35% or higher. The inhibitor of body fat percentage increase of the present invention is effective, on the other hand, irrespective of the body fat percentage.

The agent lowering body fat percentage or the inhibitor of body fat percentage increase of the present invention is effective by intake of soybean 7S protein in an amount of 3 g or larger, preferably 5 g or larger per day, but an amount of 15 g or smaller per day is sufficient in the light of the effect and for avoiding of unbalance of nutrients.

The agent according to the present invention may be formulated into a composition for oral administration containing obtained soybean 7S protein as an active ingredient. For example, according to the method known in the art, it can be formulated into the form of powder, tablet, or granule, etc. and it can be used for various foods. Materials used for various foodstuffs and additives may be appropriately added together.

Soybean 7S protein used as the active ingredient of the present invention is highly safe to permit the protein to be used for edible compositions. The amount of blending in the food and the amount of intake are not particularly restricted, and the protein may be directly taken or may be added in the food as dietary therapy.

The agent of the present invention may be provided without any restriction in any form such as tablets, beverage, yogurt, rice cracker, bread and jelly. A beverage containing 1 to 10% of 7S protein may be prepared by mixing soybean 7S protein with sugar, water, a stabilizer and fruit juice, etc. adjusting the pH at 3.5 to 4.5, homogenizing, adding flavors etc., heating, and cooling. A jelly-like food may be prepared by combining soybean 7S protein with a gelling agent. A yogurt-like food containing 1 to 15% of 7S protein may be prepared by mixing soybean 7S protein with creamy yogurt, water, sugar and gelling agent etc., adjusting the pH at 3.5 to 4.5, and sterilizing by heating. Rice crackers may be prepared by slowly adding water to a mixture of 30% or more of soybean 7S protein, a starchy substance, seasoning, etc. to obtain a dough, then kneading and dividing the dough, baking at 120 to 300°C by sandwiching between two plates, followed by drying. Bread is produced by mixing 2 to 18% of soybean 7S protein relative to wheat flour with concomitant use of phospholipid containing 45% or more of phosphatidylcholine.

While the effectiveness of the present invention is shown below with reference to examples, the technical spirit of the present invention is by no means restricted to these examples.

The assay methods mainly used in the present invention are as follows.

* SDS-polyacrylamide gel electrophoresis: the protein was assayed by the method of Laemmli (Nature, 227, 680 (1970)) using a gradient gel in the concentration of 10% to 12%. The applied quantity of the protein was 10 μg.
* Phytic acid: Phytic acid was measured according to the method of Alii Mohamed (Cereal Chemistry 63, 475-478, 1986).
* Content of the oil component extracted with chloroform/methanol: A mixed solution of chloroform/methanol (2:1 in volume ratio) about 50 times as a dry sample was added to the dry sample, and the fraction extracted at 160°C was weighed and determined the content of the oil component extracted with chloroform/methanol.
* Purity (SPE standard): The area of the electrophoresis pattern obtained by SDS-polyacrylamide gel electrophoresis was measured with a densitometer, and the purity (SPE standard) was measured as area ratio to the total area of the fraction. The 7S globulin content refers to the total content of $\alpha$, $\alpha'$ and $\beta$ subunits. Although the purity may be measured as a corrected purity by taking the amount of the oil body-associating protein into consideration, it was measured according to SPE standard in the present invention.
* Corrected purity: The sample also contains the oil body-associating protein in a quantity 10 times as much as the weight of the chloroform/methanol extract in addition to 7S globulin. Therefore, the purity relative to the total protein is calculated from the purity (A%) of the sample based on SPE standard;

Corrected purity (%) = (100(%) - content of the oil component extracted with chloroform/methanol (%) $\times$ 10) $\times$ A

(%)/100

* Body fat percentage: The body fat percentage was calculated by measuring the electric resistance of the body. Practically, the body fat percentage was measured using "8-electrode body fat measuring apparatus BC-118" manufactured by Tanita Corp.

Production Example 1 (Preparation of high purity-low phytic acid 7S protein)

10 parts by weight of extracting water at 40°C was added to 1 part by weight of low denaturation defatted soybean at 40°C, and the solution was adjusted to pH 5.3 with hydrochloric acid. Phytase (Phytase Novo L, manufactured by Novo Industries) corresponding to 8 units per protein weight was added to the solution, which was processed for extraction of the protein and enzyme reaction at 40°C for 30 minute to obtain an enzyme-treated and extracted slurry. The enzyme-treated and extracted slurry was cooled to about 25°C followed by adjusting to pH 6.1 with hydrochloric acid, and separated by centrifugation with a batch type centrifuge (1,200 G). The soluble fraction was definitely separated from the insoluble fraction by centrifugation. The temperature of the solution during centrifugation was about 25°C. Subsequently, the soluble fraction was adjusted to pH 4.9 with hydrochloric acid, and a precipitated curd was obtained by centrifugation. The precipitated curd was diluted with water (4 times in weight), and washed with 10 times as much water as the weight of the protein followed by neutralization with sodium hydroxide. Phytase-treated soybean 7S protein was obtained by spray drying after sterilization at 140°C for 15 seconds.

The purity (SPE standard) of high purity soybean 7S protein thus obtained was 95%. It was confirmed that phytic acid was almost completely decomposed and removed since the content of phytic acid in the solid content was 0.05%. On the other hand, the content of oil component extracted with chloroform/methanol in the protein was 0.5%. In addition, it was suggested to be high purity soybean 7S protein containing substantially small content of impurities with a combined content of sulfur-containing amino acids of cystine and methionine of 12 mg/g protein as compared with the content of 5 mg/g in original purified 7S protein.

Production Example 2 (Preparation of 7S protein with high purity low phytic acid, 2)

Water was added to defatted soybean in a weight ratio of 1:10. The mixed solution was stirred for 1 hour while occasionally controlling the pH at 7.0. The mixture was centrifuged (4,000 rpm, 20°C, 10 minutes), and the supernatant was adjusted to pH 6.0. Phytase (phytase Novo L: manufactured by Novo Industries) was added in a proportion of 0.2% per protein, and reacted at 40°C for 1 hour. The pH of the reaction solution was readjusted at 6.0 and centrifuged (4,000 rpm, 20°C, 10 minutes). The supernatant was adjusted to pH 5.0, followed by centrifugation (4,000 rpm, 4°C, 10 minutes). The precipitate was collected and water was added. The solution was neutralized at pH 7.0, spray-dried and sterilized to obtain low phytic acid soybean 7S protein.

The purity (SPE standard) of high purity-low phytic acid 7S protein thus obtained was shown to be 88%. The phytic acid content in the solid content was 0.05%, by which phytic acid was confirmed to be almost completely decomposed and removed. On the other hand, the content of the oil component extracted with chloroform/methanol was 1.0%. In addition, it was suggested to be high purity soybean 7S protein containing substantially small content of impurities with a combined content of sulfur-containing amino acids of cystine and methionine of 16 mg/g protein as compared with the content of 5 mg/g in original purified 7S protein.

Production Example 3 (Preparation of low phytic acid 7S protein)

Water was added to defatted soybean in a weight ratio of 1:10. The mixed solution was stirred for 1 hour while occasionally controlling the pH at 7.0. The mixture was centrifuged (4,000 rpm, 20°C, 1 minutes), and the supernatant was adjusted to pH 6.4. The supernatant was allowed to stand at 4°C overnight, centrifuged (4,000 rpm, 4°C, 10 minutes) and the precipitate was discarded. The supernatant was adjusted to pH 4.5, centrifuged (4,000 rpm, 4°C, 10 minutes) and the precipitate was collected as 7S protein curd.

Water 4 times as much as the weight of 7S protein curd was added, adjusted the pH at 6.0. Phytase (phytase Novo L: manufactured by Novo Industries) was added in a proportion of 0.2% per protein, and reacted at 40°C for 1 hour. The pH of the reaction solution was adjusted at 5.0, and the whey fraction was removed by centrifugation (4,000 rpm, 20°C, 10 minutes) to obtain low phytic acid 7S protein curd. Water was added to low phytic acid 7S protein curd, neutralized to pH 7.0, sterilized, and spray-dried to obtain low phytic acid 7S protein. The purity (SPE standard) of high purity-low phytic acid 7S protein thus obtained was 80%. The phytic acid content in the solid content was 0.05%, which confirmed that phytic acid was almost completely decomposed and removed by phytase treatment. The content of oil component extracted with chloroform/methanol was 2.8%. On the other hand, it was suggested to contain a lot of impurities, since the combined content of sulfur-containing amino acids of cystine and methionine was 25 mg/g protein as compared with the content of 5 mg/g in original purified 7S protein.

Test Example 1 [Body fat percentage-lowering effect]

32 parts of high purity low-phytic acid 7S protein prepared in Production Example 1, and 68 parts of maltose powder (Finetose: manufactured by Hayashibara Shoji, Inc.) were mixed, 6 parts of water and 14 parts of ethanol were added, and kneaded. The mixture was dried for 10 hours in a drier at 60°C, and put through a 1 mm mesh sieve. After the treatment 3 parts of sugar ester (trade name: DK-ester, manufactured by Dai-Ichi Kogyo Seiyaku Co., Ltd.), 2 parts of fruit juice powder (manufactured by Ogawa & Co., Ltd.) and 1 part of citric acid was added to the 94 parts of the above mixture, and tablets (1.7 g/tablet) containing 0.5 g of high purity-low phytic acid 7S protein were obtained using a tablet machine.

Six tablets each were taken before breakfast and dinner every day for 4 weeks (12 tablets per day which corresponds to about 5 g of 7S globulin), and the body fat percentage was measured before, and 2 and 4 week after the start of intake using an impedance body composition measuring apparatus (device name: BC-118, manufactured by Tanita Corp.).

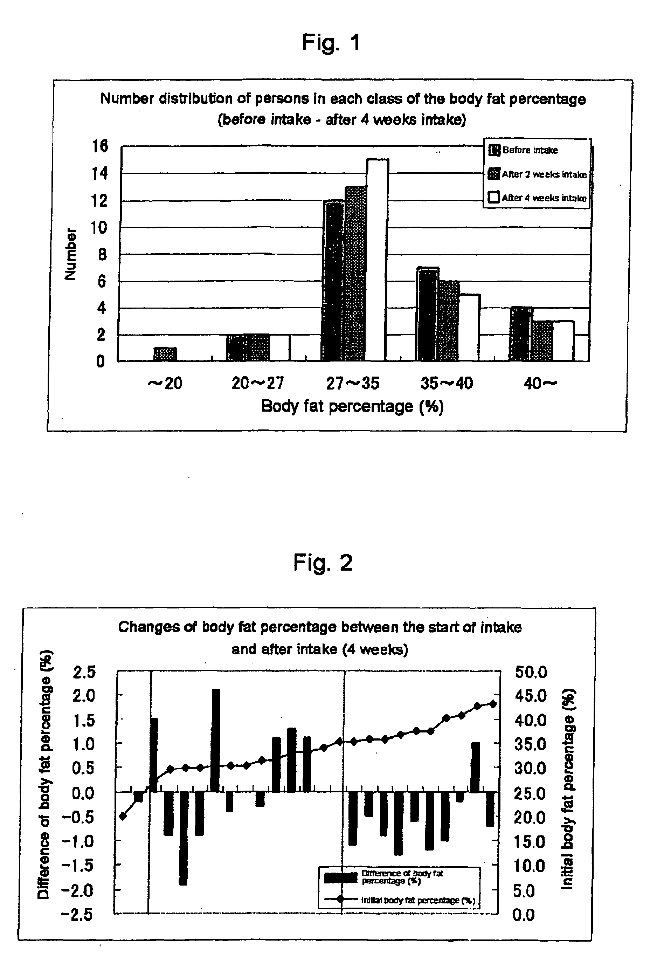

The results are shown in Table 1. It was shown that the body fat percentage was significantly lowered by taking about 5 g of soybean 7S globulin every day. This tendency is particularly remarkable for a person having a high initial body fat percentage. This result shows that 7S globulin exhibits not only a fat removal effect, but also an excellent body fat percentage-lowering effect in cooperation with homeostasis in the body. Fig. 1 shows number distribution of persons in each class of the body fat percentage (from before intake to 4 weeks). Fig. 2 shows the difference between the initial body fat percentage and the body fat percentage after 4 weeks intake.

Table 1

| Change of body fat percentage/ amount of body fat/ weight by intake of test food | | | |
|---|---|---|---|
| | Initial body fat percentage, less than 35% (n = 14) | | |
| | Before intake | 2 Weeks intake | 4 Weeks intake |
| Body fat percentage (%) | 29.6 ± 3.8 | 29.4 ± 4.3 | 29.8 ± 4.1 |
| Amount of body fat (kg) | 15.1 ± 3.3 | 15.2 ± 3.6 | 15.3 ± 3.4 |
| Weight (kg) | 50.7 ± 6.5 | 51.0 ± 6.4 | 51.0 ± 6.4 |

Table 1 (continued)

| Change of body fat percentage/ amount of body fat/ weight by intake of test food | | | |
|---|---|---|---|
| | Initial body fat percentage, no less than 35% (n = 11) | | |
| | Before intake | 2 Weeks intake | 4 Weeks intake |
| Body fat percentage (%) | $38.2 \pm 2.9$ | $37.8 \pm 3.3$ | $37.6 \pm 3.2$ * |
| Amount of body fat (kg) | $23.8 \pm 4.2$ | $23.5 \pm 4.4$ | $23.4 \pm 4.4$ |
| Weight (kg) | $61.9 \pm 6.5$ | $61.8 \pm 6.5$ | $61.8 \pm 6.4$ |

Numerical value: mean value $\pm$ standard deviation
\* Significant difference as compared with the value before intake (paired t-test, $p < 0.05$)

Similar tests were performed with 7S protein containing a high percentage of chloroform/methanol extracted fractions obtained in Production Example 3. The results showed a similar tendency for lowering the body fat percentage to the test above. However, the protein was poorer in flavor than that obtained in Production Example 1, and test objects complained it to be hardly taken for a long period of time.

Test Example 2 [Inhibiting effect on body fat percentage increase]

To 100 parts of high purity low phytic acid 7S protein prepared in Production Example 2, 14 parts of water, 6 parts of citric acid and 80 parts of ethanol were added and kneaded. After drying the mixture for 10 hours in a dryer at 50°C, the powder obtained was put through a 1 mm mesh sieve. 56.2 parts of the processed powder was mixed with 33.2 parts of maltose powder (Finetose: manufactured by Hayashibara Shoji, Inc.) and 10.6 parts of powdered sugar, 40 parts of 80% ethanol was added and kneaded. After dried at 50°C for 10 hours in a dryer, the mixture was put through a 1 mm mesh sieve. After the treatment, 3 parts of sugar ester (trade name: DK-ester, manufactured by Dai-Ichi Kogyo Seiyaku Co., Ltd.), 2 parts of fruit juice powder (manufactured by Ogawa & Co., Ltd.) and 0.5 parts of perfume were added to 94.5 parts of the mixture above to obtain tablets (1.5 g/tablet) containing 0.75 g of high purity low phytic acid 7S protein using a tablet machine. As a placebo food, tablets (1.5 g/tablet) mainly composed of carbohydrates were prepared. In the tablet, high purity low phytic acid 7S protein was substituted with sugars, dextrin and starch, and sour flavor was controlled by decreasing the amount of citric acid. Composition of the test food and the placebo food are shown in Table 2.

Table 2

| Composition of test food and placebo food | | | |
|---|---|---|---|
| | Test food (1.5 g/tablet) | | Placebo food (1.5 g/tablet) |
| Soybean 7S protein | 50.0% | Maltose | 50.0% |
| Maltose | 31.5% | Dextrin | 33.5% |
| Powdered sugar | 10.0% | Corn starch | 10.0% |
| Fruit juice powder | 2.0% | Fruit juice powder | 2.0% |
| Perfume | 0.5% | Perfume | 0.5% |
| Citric acid | 3.0% | Citric acid | 1.0% |
| Sugar ester | 3.0% | Sugar ester | 3.0% |

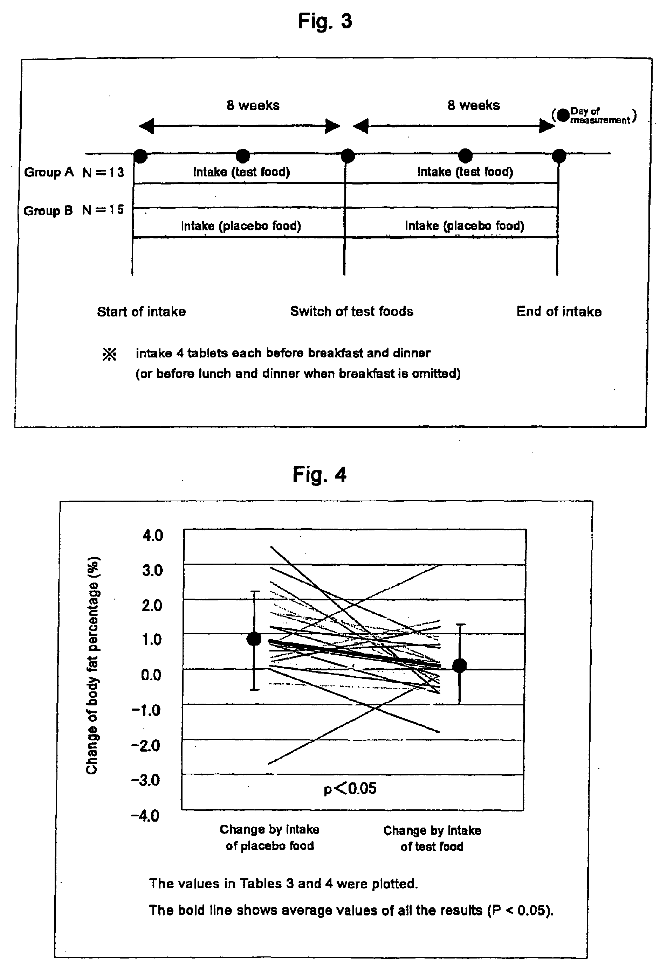

Test objects were 28 persons, and test period was 16 weeks. Using double blinded test and cross-over method, the objects were fed the test food containing 7S globulin for 8 weeks and placebo food mainly composed of carbohydrates for another 8 weeks. Normally, four tablets each, 8 tablets in total (about 5 g of 7S globulin), were taken before the breakfast and dinner (or before lunch and dinner when breakfast was omitted) everyday. The body fat percentage was measured with the impedance body composition measuring apparatus (BC-118/ manufactured by Tanita Corp.) before the start of intake, at week 4, 8 and 12, and week 16 at the end of intake. The body fat percentage was measured at the same time of lunch break or evening. The test was applied after urination before the meal so as to diminish the effect of water in the body. The test method is illustrated in Fig. 3.

The results are shown in Table 3 (group A) and Table 4 (group B). In the test results (Fig. 4) combining group A and group B, the body fat percentage was increased during the period of intake of the placebo food ($p < 0.01$), and average increase $\pm$ standard deviation was $0.8 \pm 1.4\%$. On the other hand, the increase of the body fat percentage was inhibited during the period of intake of the test food, and average decrease $\pm$ standard deviation was $0.1 \pm 1.1\%$.

This decrease was significant as compared with the increase during the period of intake of the placebo food (p < 0.05). It was shown that the increase of the body fat percentage can be significantly inhibited by taking about 5 g of soybean 7S protein every day.

Table 3

| Change of body fat percentage (group A) | | | | |
|---|---|---|---|---|
| | Body fat percentage (%) | | | |
| Test object (n = 13) | initial value | Δ1 | Δ2 | Δ2-Δ1 |
| | | Change by intake of test food | Change by intake of placebo food | |
| A1 | 23.2 | 1.2 | 0.2 | -1.0 |
| A2 | 23.3 | 0.8 | 2.9 | 2.1 |
| A3 | 24.9 | -2.7 | 0.7 | 3.4 |
| A4 | 27.4 | 0.8 | 2.2 | 1.4 |
| A5 | 28.8 | -0.7 | 0.7 | 1.4 |
| A6 | 29.0 | -0.7 | 3.5 | 4.2 |
| A7 | 29.4 | -0.2 | -2.7 | -2.5 |
| A8 | 29.9 | 0.0 | 0.7 | 0.7 |
| A9 | 31.2 | 1.4 | 0.3 | -1.1 |
| A10 | 32.5 | -0.9 | -3.1 | -2.2 |
| A11 | 33.6 | 0.4 | 1.4 | 1.0 |
| A12 | 42.8 | 0.1 | 0.0 | -0.1 |
| A13 | 42.8 | -0.2 | 0.2 | 0.4 |
| Average ± standard deviation | 30.7 ± 6.3 | -0.1 ± 1.1 | 0.5 ± 1.9 | 0.6 ± 2.0 |
| Significance of difference between test foods | |- P = 0.31 -| | | | |

Table 4

| Change of body fat percentage (group B) | | | | |
|---|---|---|---|---|
| | Body fat percentage (%) | | | |
| Test object (n = 15) | initial value | Δ1 | Δ2 | Δ2-Δ1 |
| | | Change by intake of test food | Change by intake of placebo food | |
| B1 | 23.8 | -0.4 | -0.6 | -0.2 |
| B2 | 23.8 | 1.6 | 0.9 | -0.7 |
| B3 | 24.2 | 0.2 | 0.9 | 0.7 |
| B4 | 24.9 | 1.2 | -0.2 | -1.4 |
| B5 | 26.7 | 2.2 | 0.2 | -2.0 |
| B6 | 26.8 | 1.9 | -0.3 | -2.2 |
| B7 | 27.6 | 1.3 | 1.5 | 0.2 |
| B8 | 27.6 | 1.6 | 0.2 | -1.4 |
| B9 | 28.1 | 2.5 | -0.4 | -2.9 |
| B10 | 30.7 | 0.5 | 0.7 | 0.2 |
| B11 | 31.4 | 0.7 | 3.0 | 2.3 |
| B12 | 33.7 | 1.2 | 0.6 | -0.6 |
| B13 | 35.3 | 0.7 | 0.2 | -0.5 |
| B14 | 41.4 | 0.0 | -1.8 | -1.8 |

Table 4   (continued)

| Change of body fat percentage (group B) | | | | |
|---|---|---|---|---|
| | | Body fat percentage (%) | | |
| Test object (n = 15) | initial value | Δ1 | Δ2 | Δ2-Δ1 |
| | | Change by intake of test food | Change by intake of placebo food | |
| B15 | 42.1 | 0.1 | -0.5 | -0.6 |
| Average ± standard deviation | 29.9 ± 5.9 | 1.0 ± 0.9 | 0.3 ± 1.1 | -0.7 ± 1.3 |
| Significance of difference between test foods | |- $\qquad$ P < 0.05 $\qquad$ -| | | |

The present invention shows the effectiveness of the body fat percentage-lowering ability or body fat percentage increase inhibiting ability of low phytic acid 7S protein. It was shown that intake of about 5 g of soybean 7S protein every day is effective for lowering the body fat percentage or inhibiting increase of the body fat.

Industrial Applicability

As above described, the present invention shows that the effect for lowering the body fat percentage or inhibiting increase of the body fat can be exhibited by intake of agents or foods containing soybean 7S protein as an active ingredient.

**Claims**

1.   An agent lowering body fat percentage or an inhibitor of body fat percentage increase containing soybean 7S protein as an active ingredient.

2.   The agent lowering body fat percentage according to Claim 1, for overweight persons with a body fat percentage of 30% or more.

3.   The agent lowering body fat percentage or the inhibitor of body fat percentage increase according to any one of Claims 1 and 2 containing 2.5% or less of polar lipids in a solid content of soybean 7S protein.

4.   The agent lowering body fat percentage or the inhibitor of body fat percentage increase according to any one of Claims 1 and 2 containing 1.2% or less of phytic acid in a solid content of soybean 7S protein.

5.   Foodstuffs containing the agent lowering body fat percentage or the inhibitor of body fat percentage increase according to Claim 1.

## Fig. 1

Number distribution of persons in each class of the body fat percentage (before intake - after 4 weeks intake)

## Fig. 2

Changes of body fat percentage between the start of intake and after intake (4 weeks)

## Fig. 3

8 weeks — 8 weeks — ( ● Day of measurement )

Group A N = 13    Intake (test food)    Intake (test food)

Group B N = 15    Intake (placebo food)    Intake (placebo food)

Start of intake    Switch of test foods    End of intake

※ intake 4 tablets each before breakfast and dinner
(or before lunch and dinner when breakfast is omitted)

## Fig. 4

Change of body fat percentage (%)

$p < 0.05$

Change by Intake of placebo food    Change by Intake of test food

The values in Tables 3 and 4 were plotted.

The bold line shows average values of all the results ($P < 0.05$).

# EP 1 541 160 A1

<table>
<tr><td colspan="2" align="center">**INTERNATIONAL SEARCH REPORT**</td><td>International application No.<br>PCT/JP03/05305</td></tr>
</table>

**A.  CLASSIFICATION OF SUBJECT MATTER**
Int.Cl$^7$  A61K35/78, 38/02, A61P3/04, 3/06, A23L1/20

*According to International Patent Classification (IPC) or to both national classification and IPC*

**B.  FIELDS SEARCHED**

Minimum documentation searched (classification system followed by classification symbols)
Int.Cl$^7$  A61K35/78, 38/02, A61P3/04,3/06, A23L1/20

Documentation searched other than minimum documentation to the extent that such documents are included in the fields searched

Electronic data base consulted during the international search (name of data base and, where practicable, search terms used)
CA(STN), BIOSIS(STN), MEDLINE(STN), EMBASE(STN), JSTPLUS(JOIS),
JMEDPLUS(JOIS)

**C.  DOCUMENTS CONSIDERED TO BE RELEVANT**

| Category* | Citation of document, with indication, where appropriate, of the relevant passages | Relevant to claim No. |
|---|---|---|
| X<br>Y | Tatsuya MORIYAMA et al., "Mouse ni okeru Daizu 7S Globulin (β-Conglycinin) Shoku ni yoru Kessei Triglyceride Teika Sayo; Mouse ni okeru Daizu 7S Globulin (β-Conglycinin) Shoku ni yoru Kanzo Shishitsu Taisha Kanren Koso no Kassei Henka", Nihon Nogei Kagaku Taikai Koen Yoshishu, 05 March, 2002 (05.03.02), Vol.2002, page 108, 3-3Da12,13 | 1,2,5<br>3,4 |
| X | Toshiaki AOYAMA et al., Reduction by Phytate-reduced Soybeanβ-Conglycinin of Plasma Triglyceride Level of Young and Adult Rats, Biosci.Biotech. Biochem., 2001, Vol.65, No.5, pages 1071 to 1075 | 1-5 |

| ☒ | Further documents are listed in the continuation of Box C. | ☐ | See patent family annex. |
|---|---|---|---|

| *    Special categories of cited documents:<br>"A"   document defining the general state of the art which is not considered to be of particular relevance<br>"E"   earlier document but published on or after the international filing date<br>"L"   document which may throw doubts on priority claim(s) or which is cited to establish the publication date of another citation or other special reason (as specified)<br>"O"   document referring to an oral disclosure, use, exhibition or other means<br>"P"   document published prior to the international filing date but later than the priority date claimed | "T"   later document published after the international filing date or priority date and not in conflict with the application but cited to understand the principle or theory underlying the invention<br>"X"   document of particular relevance; the claimed invention cannot be considered novel or cannot be considered to involve an inventive step when the document is taken alone<br>"Y"   document of particular relevance; the claimed invention cannot be considered to involve an inventive step when the document is combined with one or more other such documents, such combination being obvious to a person skilled in the art<br>"&"   document member of the same patent family |
|---|---|

| Date of the actual completion of the international search<br>16 June, 2003 (16.06.03) | Date of mailing of the international search report<br>01 July, 2003 (01.07.03) |
|---|---|
| Name and mailing address of the ISA/<br>    Japanese Patent Office | Authorized officer |
| Facsimile No. | Telephone No. |

Form PCT/ISA/210 (second sheet) (July 1998)

11

**EP 1 541 160 A1**

## INTERNATIONAL SEARCH REPORT

| | International application No. |
|---|---|
| | PCT/JP03/05305 |

C (Continuation).   DOCUMENTS CONSIDERED TO BE RELEVANT

| Category* | Citation of document, with indication, where appropriate, of the relevant passages | Relevant to claim No. |
|---|---|---|
| P,X | Makoto KITO, "Arushu no 'Daizu Tanpaku' wa Chusei Shibo o Haijo suru (Kitai sareru Himan Koka)", Japan Food Science, 05 September, 2002 (05.09.02), Vol.41, No.9, pages 92 to 94, Fig. 4 | 1-5 |
| P,X | JP 2002-238442 A (Fuji Oil Co., Ltd.), 27 August, 2002 (27.08.02), (Family: none) | 1-5 |
| Y | JP 2002-114694 A (Fuji Oil Co., Ltd.), 16 April, 2002 (16.04.02), (Family: none) | 1-5 |
| Y | OKITA, Takuo et al., Effects of Dietary Soybean Globulins on Plasma and Liver Lipids and on Fecal Excretion of Neutral Sterols in Rats, J.Nutr.Sci. Vitaminol., 1981, Vol.27, No.4, pages 379 to 388 | 1-5 |
| Y | EP 380348 A2 (ALKO LTD.), 09 August, 1990 (09.08.90), & WO 90/08476 A1          & CA 2044240 A & FI 913538 A            & CN 1048310 A & IN 170809 A            & JP 4-503002 A | 1-5 |
| Y | EP 10-203994 A (Ichimaru Pharcos Co., Ltd.), 04 August, 1998 (04.08.98), (Family: none) | 1-5 |
| Y | RITTER, M.A et al., In vitro Digestibility Phytate-reduced and Phenolics-reduced Soy Protein Isolates, J.Foog.Sci., 1987, Vol.52, No.2, pages 325 to 327 | 1-5 |
| Y | JP 9-23822 A (Nichimo Co., Ltd.), 28 January, 1997 (28.01.97), (Family: none) | 1-5 |
| Y | JP 8-173052 A (Morinaga & Co., Ltd.), 09 July, 1996 (09.07.96), (Family: none) | 1-5 |
| Y | JP 2000-204369 A (Fuji Oil Co., Ltd.), 25 July, 2000 (25.07.00), (Family: none) | 1-5 |
| Y | SAMOTO M. et al., Biosci.Biotech.Biochem., "Oil Body Kaigo Tanpaku-shitsu to Kyokusei Shishitsu no Joho ni yoru Bunri Daizu Tanpaku-shitsu no Off Flavor no Teigen", 1998, Vol.62, No.5, pages 935 tp 940 | 1-5 |
| Y | Tadashi OGAWA et al., "Tei Allergen Daizu Tanpaku-shitsu Riyo Shokuhin no Kaihatsu ni Kansuru Kenkyu", Daizu Tanpakushitsu Kenkyukai Kaishi, 1994,Vol.15, pages 104 to 108 | 1-5 |

Form PCT/ISA/210 (continuation of second sheet) (July 1998)

12

**INTERNATIONAL SEARCH REPORT**

International application No.

PCT/JP03/05305

C (Continuation). DOCUMENTS CONSIDERED TO BE RELEVANT

| Category* | Citation of document, with indication, where appropriate, of the relevant passages | Relevant to claim No. |
|---|---|---|
| Y | Kengo Ishihara et al., "Daizu Tanpaku-shitsu Bunkai Peptido no Tiashibo Chikuseki Seigyo Koka ni Kansuru Kenkyu", Daizu Tanapakushitsu Kenkyukai Kaishi, 1997, Vol.18, pages 96 to 100 | 1-5 |
| Y | Kiyoharu TAKAMATSU , "Daizu to Shibo Taisha", Japan Oil Chemists' Society Kansai Shibu Kaki Seminar 18-19. July, 2000 (18-19.07.00), Vol.40th, pages 45 to 55 | 1-5 |

Form PCT/ISA/210 (continuation of second sheet) (July 1998)